# EUROPEAN PATENT APPLICATION

(11) **EP 3 978 631 A1**
(43) Date of publication of application: **06.04.2022**
(21) Application number: 20812509.6
(22) Date of filing: 08.05.2020
(51) Int. Cl.: C12Q 1/6886

(54) **SQUAMOUS CELL CANCER DIAGNOSTIC OR PROGNOSIS PREDICTION MARKER AND USE THEREOF**

(30) Priority: 28.05.2019 KR 20190062423
(71) Applicant: Industry-University Cooperation Foundation Hanyang University, Seoul 04763 (KR)
(72) Inventor: NAM, Jin-Wu, Seoul 06009 (KR); YOU, Bo-Hyun, Seoul 04763 (KR)
(74) Representative: dompatent von Kreisler Selting Werner - Partnerschaft von Patent- und Rechtsanwälten mbB
(86) International application number: PCT/KR2020/006063
(87) International publication number: WO 2020/242080

(57) **Abstract**

The present invention relates to a squamous cell carcinoma diagnostic or prognosis prediction marker and the use thereof and, more particularly, to the use of a long non-coding RNA as a marker composition for diagnosing or predicting the prognosis of squamous cell carcinoma, a diagnostic composition, diagnostic information-providing method and the like, by means of the correlation between an expression of the long non-coding RNA and squamous cell carcinoma pathogenesis. The inventors discovered that the expression pattern of a long non-coding RNA is related to cancer pathogenesis, investigated the correlation between the expression of a specific long non-coding RNA and squamous cell carcinoma pathogenesis and thus confirmed that the long non-coding RNA can be a squamous cell carcinoma diagnostic or prognosis prediction marker. Therefore, the marker according to the present invention can assist management and treatment of a cancer patient by means of diagnosing or predicting the prognosis of squamous cell carcinoma.

<110>     INDUSTRY-UNIVERSITY COOPERATION FOUNDATION HANYANG UNIVERSITY

<120>     Markers for diagnostic or prognosis predictive of squamous cell
          carcinoma and uses thereof

<130>     PCT2020-008

<150>     KR 2019/0062423
<151>     2019-05-28

<160>     19

<170>     KoPatentIn 3.0

<210>     1
<211>     748
<212>     RNA
<213>     Homo sapiens (RP11-114H23.1-001 ENST00000552856.1)

<400>     1
gtggaagtga ggaagaggga gagctcctgg gcaccagaat gaatggggag ggtcctttag        60

ggcactgtga ggccattagc ttttactcct actgagactg gacatcccag gagagtttca       120

gagcagagaa gtgctatgac tgctcttgaa gtttaagagg accaggtggc tggctttgct       180

gtgtgtcaga gaacaggagc agaaacaggg agaccagctg ggaggtgact gcaaaagtcc       240

aggtgagagg cgatggagct agtgaggagt gccggaggcc tggtgtattc tgacggtgcc       300

aggacctgtg ataggtgctg agagagatga ggaactgctc agatcaccag ctgcttcagg       360

ttaccagccc acacctgtct gtctcacctc tgacaacctt ggtaaagaaa ccaaggctta       420

gagacctgct cacagtcgtt tcgtgaggga atgcaacatc tgggtctagg gtccaaatct       480

atctctgctg tgaagaggaa ctcatacagg agatctacat gattaccaat tcttggcctt       540

gcagtggagg atattaattt taagtacctg tgaaatgaag ttcttaaaac ctaagattcc       600

ccaaaactcc agttatcaac aaggttatat aggaatcaaa gcgaggcgtt tggagtagat       660

gccgagatca cctctccctc acacctctgc tgaccccagc tacctgcaat cctgccagga       720

ccattctgga ggtgaggtga tccttttt                                          748


<210>     2
<211>     712
<212>     RNA
<213>     Homo sapiens (RP11-114H23.1-003 ENST00000552466.2)

<400>     2
actacttctc cagattctga atgttctaca tctgggcaca agattaccgt tctttcattg        60

taccatctca ggctcagtca ttgttactta agcaggccac catgttgcgg gactggtggg       120

gatgagcttg caggctcagt tgtcagtgtg agaactttcc ctttcctatg accttccaga       180

tgaggaactg ctcagatcac cagctgcttc aggttaccag cccacacctg tctgtctcac       240

ctctgacaac cttgatataa attaaacaat gaataagaac aaaaacaatt taaaactgcg       300

taatggtcca aaccaccatg gatggtgttc tgacgatgat acacggggtg aggtctgact 360

catgagccgc tgtccgtgat gacttgacat tctcaaaagg aaatcccgaa tgttctcact 420

gcatgctgct gcaggccctc atggcagggc atccacacca ctggctcagg tgctggttgc 480

tccagaatct ctcttcagct ggcctttttc tactgaactg tctgtgagaa catcactgca 540

ggcacggttt tctccaacca taattgctgc tgccaaagcc acctcggact tgggaaaggg 600

aaacaacact ccactagaga gcgtagaatc ttagaattca tctacttcat tctcctcctg 660

gtactctact ttcttctaca acgtcccagc cgagctgagg tctgagctgt gc 712

<210> 3
<211> 2160
<212> RNA
<213> Homo sapiens (HERES.1)

<400> 3
ggcaaccagc ttgcgtccgt ttccatgttg tgggagattt gttctttcgc tctttgtggt 60

aaatcttgct actgctcact ctttgagtcc acactgcttt tatgagctgt aacactcacc 120

gggaaggtct gcagcttcaa tcctgaagcc agcgaggcca ccagcccacc gggaggaacg 180

aacaactcca gacgcaccgc cttaagagct gtaacactca ccgcgaaggt ctgcagcttc 240

actcctgagc cagcgagacc acgaacccac cagaagaaag aaactccgaa cacatccgaa 300

caccagaagg aacaaactcc agacgcgcca actgtaacac tcaccgcgag gatccgtggc 360

ttcattcttg aagtcagtga gaccaagaac ccaccaattc cggacacact gtgagtcagg 420

atcagtctta ttatagctaa ttagagcaac cccattgcta aagagtttag agcctgggtc 480

agacttcatg gtagcaaagc aatgttttat tgtaatagtg taattaggaa tgtgtggcaa 540

tattttaacc cctcctccca agagttccac aatgctttct taacatgttt ataaatattt 600

gcttttgtgc cattttaggg gagctttccg tacaaacatt tggcttttaa tgagtcatcc 660

acactctgat tcatacccat aagcttcctg ctttgttatc agtgaatatt gggctcagta 720

actgccttac ttttataatt tacatgaatg aatatggaaa aggtggtgga ggtagaaaga 780

catattcagg taaatataaa aacaccctgg tttctttaca agagattgac aaatacggcc 840

caatacccta tcatttaaaa aaatagctgc tgccccactc ctgtgccaaa atctgctcca 900

agggatccta tcctagtcag aaggctgtac tctgaatgct gatattagct acaacaccaa 960

ggcttcccac tatacaaact ggatcctcaa agtcatccac accaaatgaa ctctctattc 1020

tttctcccag tagcctgaga actgggttta ccagccttgg cccttccttt ccttctagcc 1080

tcaaaacagg atgggaacag agtctttggc tgtgagaatg ggctcaataa caaaataaag 1140

atgacagaag agtcagtgac ttcaatgaca gagcgataga aattaccaat ataaacggag 1200

agaaaagaga ttttttaaaa atgaacaaat ccccaaggac ctgtgagaca ataactgtgt 1260

taggttttct aaacttacaa gtgtttttat ttatatatgg taagcaacta tgcattattt 1320

gtgtcagaat tattttttgt atatatttaa ggtatacaac atgatgtctc aacatactta 1380

tatgtagtga aatggttact acagtcacca cacttattgt ctcacaggtt attgtcttca 1440

gaaggagagt aaaaagaatg ctgggtagaa gaaatacctg aagaaataat ggcccaacct 1500

caaatttggt gaaagacata aacttacaga ttcaagaaac tgagtgttcc ccaaacagaa 1560

taaactttaa agaatccaca ctcaaacaca tcataatcaa acttctgaaa actaacaaca 1620

aagaaaaact taaaatcagc aagagagaaa ccacacatta tcaactgatg aaaagtgact 1680

caagtgacag cagatttata atcagaaact tgaaggacag aaggaagagg caaaacattt 1740

tgaaagccct aaaaggaaag aactgtcaac ccagaattcg acatccagta aaaatatccg 1800

tcaagaatga atatgaagga aagctaagaa tgaatatatc ttctatttct ttgctaaatt 1860

tttctacttt ttcatttgat gtaagagtgt ttataagtgc ttatggagca tttttatgat 1920

agttggttta caatccttgt ctgagagtag ctatgtgaga tgacgtatat agtaaatggt 1980

gactgtagta accatttcac tacatataag tatgtcaaaa catgttgtac accttaaata 2040

tacacaaaaa atagctctga cacaaaaaat gcatagttgt tattaccatg tataaataaa 2100

aatacttgta agtttagaaa ataaaatcat tgtctgataa ttatctaaat aataaagtcc 2160

2160

<210> 4
<211> 6675
<212> RNA
<213> Homo sapiens (HERES.2)

<400> 4
ggcaaccagc ttgcgtccgt ttccatgttg tgggagattt gttctttcgc tctttgtggt 60

aaatcttgct actgctcact ctttgagtcc acactgcttt tatgagctgt aacactcacc 120

gggaaggtct gcagcttcaa tcctgaagcc agcgaggcca ccagcccacc gggaggaacg 180

aacaactcca gacgcaccgc cttaagagct gtaacactca ccgcgaaggt ctgcagcttc 240

actcctgagc cagcgagacc acgaacccac cagaagaaag aaactccgaa cacatccgaa 300

caccagaagg aacaaactcc agacgcgcca actgtaacac tcaccgcgag gatccgtggc 360

ttcattcttg aagtcagtga gaccaagaac ccaccaattc cggacacact gtgagtcagg 420

atcagtctta ttatagctaa ttagagcaac cccattgcta aagagtttag agcctgggtc 480

agacttcatg gtagcaaagc aatgttttat tgtaatagtg taattaggaa tgtgtggcaa 540

tattttaacc cctcctccca agagttccac aatgctttct taacatgttt ataaatattt 600

gcttttgtgc cattttaggg gagctttccg tacaaacatt tggcttttaa tgagtcatcc 660

acactctgat tcatacccat aagcttcctg ctttgttatc agtgaatatt gggctcagta 720

actgccttac ttttataatt tacatgaatg aatatggaaa aggtggtgga ggtagaaaga 780

catattcagg taaatataaa aacaccctgg tttctttaca agagattgac aaatacggcc 840

caatacccta tcatttaaag taagtgccag aattgtgatg gatttgtgtt tggccttaaa 900

aaaaaataaa aataaaaata aaaacaaaaa cacttggata attctacctg atgttacatt 960

tttctttttt tttttctttt cttttttttt tttttttttt gagatggagt tttcctctgt 1020

cacccaggct ggagtgcagt gacgtgacct cagctcgctg caacctctgc ctcccagatt 1080

caagcaattc tctctcaaat tgctcagaag ctgagcaatt ctgcctcagc ttcccaaata 1140

gctgggatta caggtgcgtg ccaccaggct cagctaattt ttgtattttt agtagagatg 1200

gggtttcacc atgttggcca ggctggtctt gaactcccga cctcaggtga tacgcccgcc 1260

tcggcctccc aaagtgctgg gattacaggc atgagccact gcgcccagcc tgatgttcca 1320

tttttctaat caccaagtgt atttattagc taagggtatc ccagaaatta aattaccaac 1380

ataaaatttt acttgtgcta ttataattta gctattaaat cctaaaatta tcaactgaaa 1440

gctactggaa aaataaatac ttctctttct tttctccata ttgaaagtaa agagaatgag 1500

gatccttagc taagatttta attaaatctt ttctgagcca gaattgcaaa gcttttcttt 1560

gtataaataa ggactcgagt tataaagcat agaactagca atgagtctat cttggtccat 1620

ttggattgct ataaaggagt acctgaggct gggtaattta taaagaaaag acgtttattc 1680

gacacatgat tctgcaggat gtacaagaag catggcacca gcatctgctc ttggtgaaaa 1740

cttcaggtgg ctttgactcc tggtggaaga agggaagtct agcttgtgca gagctcccat 1800

ggccagagaa ggaagggggg tttgaggagg tgccaggctc tttgaataga gcgagaactc 1860

actcattacc aagagaaggc accaagccat tcatgaggga tacacccctg tgacccaaac 1920

cttgcccccc ttccaacatt ggggcttaaa tttcaatgtg atgtttaggg gtacaaacat 1980

ccaaaccaca gcagagaaat acagagttct gtatttctat tcccaaaggg caaaatttgg 2040

gaggtgaatg ggtctatatg tgtgttgggg ggaagaagtt aatatgaaga aaagaaacta 2100

aagtgcttta cataataatg agaattacag attaaaaaat ctgtaatctt tccctaaaaa 2160

gggaaagaag taccatgcaa attatgaggt aagtagacta tatgacttat ttatttttat 2220

taggttagag caagaattgc aaactcaagg tcctgactcc ttgaattgca ctctatggac 2280

ccagtcatac ttcatttgca tattatatat taaatgcttt ggcatgatct ttgctccccc 2340

ttacatcctc aaaacactct ttttccattt ttcttaaaat atatgttcct ctttgtctat 2400

attttatttt tccaactttg aaaaggactt acacataaaa atggtatcac tttcttctta 2460

actgtaagaa aaatggtctc agccatacat tgatgaatgg cagctgtcat tcagcctcag 2520

tgtgaaggag acaacaatga gtggtgagga ctgtaataaa atggaaagca catgcccatc 2580

caaaagtgag cattcacttc taggaactag taaaaatggt aatagccaga tctttctatt 2640

ttttcacaag aagcctgaaa gtatttaaat attttatgac taattcaaat gctttaaaaa 2700

taagcaattt gattctagga ggccaaacta gttgctatct atgatttaaa ccatagtgtc 2760

tttcagaatt aatgtatgtc cttggcgggt gtgtgtgtgt gtgtgtgtgt gtgatcccaa 2820

aacaccaata attaccagtg aaaaatttag gagattaaat atgtgtactt taatccacat 2880

tgggggaaga gagagatata aaaggtgctg agaaccttga agtggtgccc ctcctgatct 2940

gccttctacc tcttcaacat acgtgctgtg caccatgaaa gacttgaagt gcaatggcaa 3000

tctgtgatcc tttgtggaaa cctttcccta atttgcccca gagtgaatgt ttcacacttg 3060

gattcacatt tgctgagaga ggcggaaaaa tcttagggac aattagaaca ataataacaa 3120

cattcatatt attattcaat gacagcaaca ttcatattca ttctttcatt cagcaaatat 3180

ttattgcaca cttgctatgt gctagaacta taaaatggca atgatgtgtt aggtaatttg 3240

taatcatggg gaaaaaatca gttagggaaa gacaaaactg ccttccctta tagagattat 3300

attcattgag gagacaaata ataaataaaa tgaatagtgt aacataggtt agatagtgac 3360

aagtgctatg gagaaaaaga aagtaggaaa gggtcatttc tctggtttcc tgatgtgtaa 3420

ttggaataga tatccttggt agctggtaga atctctgcag tgattccttg tactgtgggg 3480

taaaagctat gttagcaggg gagaccaaga ggaatagctg aaacaaaatt cactttctca 3540

gtaagatagt aaataagaaa gtgatattgc atcctgggaa acatggccga gattagtgct 3600

tctctttaag acctaaagga tgcagagtga tggccctcac tcatcctcat ttaattcacc 3660

tgtcaggccc ttgcaaaaat tggtctgatc ctgggggata ataaactcaa ccaaatagtt 3720

gccccaattg cttttgttat gcgagatata tctttgctag aacaaactga ttaacctggc 3780

ctcaggtacc ttgcattgat tggcaaatgt attcatttct attcctgtaa agaaagagaa 3840

acaaaagcac gggaatgaca cagactttta cagcactgag cagggctaat tctcatgccc 3900

tctgtcataa tataacctaa agggacttgg actgtctgga tactccgcac atcaacacca 3960

taacccacta tactgatgat atcatgtgtg atggttttca aatatgcaca caaatttctg 4020

attcttcttt cttcaagagg tggagcttaa ttccccttca attgagggtg agtcgggcat 4080

agtgacttgc ttctaattaa taaaatattg cagaagtgat gatgtggttt ttctaagaca 4140

ctgcaacttc ctttccttgc tctctcttga ataacatgct ctgaaggaaa tcaggtacta 4200

tgtgatgagg acattcaagc aatcctaggg agagattcat gtagaaaaga actaaggcct 4260

cctgccaaaa gccatttgag tcagccctct tggaagtaaa ccctctaccc ctagtaaagc 4320

cttcggatga ctgcagccct gggcagcatc ttgactgcaa actcatgaga gaccctgagc 4380

cacaatcacc cagctaagcc actcttaaat tcctgtccta tactgtgaga taataaatgt 4440

ttactggttt cagctgaaag gtgacagagt gggagtcatg tcaccatctc tggctgtaca 4500

cggaagaatt acagtgagtg acctttaaca caacccctta gtgctccttc ggtacccagt 4560

aaatgagggt tatcatacaa ctatgattat agagcaatag atctggctta ttcatgagac 4620

acttctgagc agtcataaat taaagcatgc atgcaaaagc ccaagatatg ccgttaacaa 4680

cactggaacc agacctcggt tttctcactt tattatctgt tctcctcttc tttggtttct 4740

ttttattttg tttttttcca tcattgaatg atggtgaagt taataatgga aaagagggag 4800

ggaagaagag aagaaaggaa gttgaatata tgaaggacca aagagaatat taccttcctc 4860

cttctcgggt gtgcttagac acaaaaccta acatccaaat aaaccaaggt gtcatttgga 4920

tctctaatgg agattgccaa gagttctgta tgagacagat tcctattaaa gtctcagaac 4980

acgttctgtg caagatcatc tctggagagc atatatgttg ggaaaccctt ttccctctgc 5040

cttccctaaa tttggtctgg aacactgttt tggggtccac acatatcctt caacccaaca 5100

caatctcaac acaagaagag atcaggtaaa atggggatca ggaaggaata tgtatccttc 5160

ttccagtcat gaatgaactt agatatcagg gggacaaatt tctgagtgtt atttctccat 5220

ggcttcccct gatgtggcct aagtgggtga ggaggcattt ttcccagccc ttatcaccca 5280

agtctttcta ttctggcccc tcagatagag acttggtaat acatcacttt tgttttggct 5340

gccctgcctg acccacctct cctctttcta acagaaaata gctgctgccc cactcctgtg 5400

ccaaaatctg ctccaaggga tcctatccta gtcagaaggc tgtactctga atgctgatat 5460

tagctacaac accaaggctt cccactatac aaactggatc ctcaaagtca tccacaccaa 5520

atgaactctc tattctttct cccagtagcc tgagaactgg gtttaccagc cttggccctt 5580

cctttccttc tagcctcaaa acaggatggg aacagagtct ttggctgtga gaatgggctc 5640

aataacaaaa taaagatgac agaagagtca gtgacttcaa tgacagagcg atagaaatta 5700

ccaatataaa cggagagaaa agagattttt taaaaatgaa caaatcccca aggacctgtg 5760

agacaataac tgtgttaggt tttctaaact tacaagtgtt tttatttata tatggtaagc 5820

aactatgcat tatttgtgtc agaattattt tttgtatata tttaaggtat acaacatgat 5880

gtctcaacat acttatatgt agtgaaatgg ttactacagt caccacactt attgtctcac 5940

aggttattgt cttcagaagg agagtaaaaa gaatgctggg tagaagaaat acctgaagaa 6000

ataatggccc aacctcaaat ttggtgaaag acataaactt acagattcaa gaaactgagt 6060

gttccccaaa cagaataaac tttaaagaat ccacactcaa acacatcata atcaaacttc 6120

tgaaaactaa caacaaagaa aaacttaaaa tcagcaagag agaaaccaca cattatcaac 6180

tgatgaaaag tgactcaagt gacagcagat ttataatcag aaacttgaag gacagaagga 6240

agaggcaaaa cattttgaaa gccctaaaag gaaagaactg tcaacccaga attcgacatc 6300

cagtaaaaat atccgtcaag aatgaatatg aaggaaagct aagaatgaat atatcttcta 6360

tttctttgct aaatttttct actttttcat ttgatgtaag agtgtttata agtgcttatg 6420

gagcattttt atgatagttg gtttacaatc cttgtctgag agtagctatg tgagatgacg 6480

tatatagtaa atggtgactg tagtaaccat ttcactacat ataagtatgt caaaacatgt 6540

tgtacacctt aaatatacac aaaaaatagc tctgacacaa aaaatgcata gttgttatta 6600

ccatgtataa ataaaaatac ttgtaagttt agaaaataaa atcattgtct gataattatc 6660

taaataataa agtcc 6675

<210> 5
<211> 583
<212> RNA
<213> Homo sapiens (RP11-1L12.3-001 ENST00000525302.1)

<400> 5
agtttgatct cagactgctg tgctagcaat cagcgagact ccgtgggcgt aggaccctcc 60

gagccagatg aagatcctga ataccaaaga gggccgctga caggtctagg agtacacttc 120

tagcacctag cagagagagg cttcactaca tcatgcttcc tgacatctct ccctttgaag 180

agcagtcaga ctcctgcttt gctcttcaga cttaatttgg gggtttaaca gccatgtgaa 240

gtgctcactc cccctttgcc ttctgccatg attgtgtgtt tcctgaggcc tccccagaag 300

ctgagaagat gcttcctgta cagcctgcag aactggatag cccagagcta ttccatgtat 360

tccatggatg ggcacatttg gaagtttggt tctaaaatct aaaagaagaa atttaagtgt 420

gctgaagata actgcaactc caaacctaac gctgtgtaat tctatgttct gccattttga 480

agcaaagtgg aaccagaagt gagaggacta tttctaggtc atgctacggt taccctgacc 540

ccagtcactc cctatgctgg tccatccaaa ctcagagcca cga 583

<210> 6
<211> 471
<212> RNA
<213> Homo sapiens (RP11-1L12.3-002 ENST00000526061.1)

<400> 6
aaactctgcc tgcttttaga tggctcctct tagaactatg catctctcct gatacttgga 60

tgcttttcct ctgactgatg aagatcctga ataccaaaga gggccgctga caggtctagg 120

agtacacttc tagcacctag cagagagagg cttcactaca tcatgcttcc tgacatctct 180

ccctttgaag agcagtcaga ctcctgcttt gctcttcaga cttaatttgg gggtttaaca 240

gccatgtgaa gtgctcactc cccctttgcc ttctgccatg attgtgtgtt tcctgaggcc 300

tccccagaag ctgagaagat gcttcctgta cagcctgcag aactgtttga atgagagagg 360

cagaagcaca tttccaatca ctgggggaac ctgagaccat tctttccaac ccattttgcc 420

aaatggatgt cctgaggaag gtgttccaag agaggcgatg tcaggatagt g 471

<210> 7

<211>     745
<212>     RNA
<213>     Homo sapiens (RP11-1L12.3-003 ENST00000531363.1)

<400>     7
agactgctgt gctagcaatc agcgagactc cgtgggcgta ggaccctccg agccaggtgc      60

gggatataat ctcgtggtgc gacgcttttt aagcccttca gaaaagcgca gtgttcgggt     120

gggagtgacc cgattttcca ggtgccgccc gtcacccctt tctttgactc agaaagggaa     180

ctccctgacc ccttgcgctt cccaagtgag gcaatgcctc gccctgcttc ggctcgcgca     240

cggtgcgcgc acccgctgac ctgcacccac tgtctggcac tccttagtga gatgaacccg     300

gtacctcaga tggaaatgca gaaatcaccc gtcttctgcg tcgctcacgc tgggagctgt     360

agaccggagc tgttcctatt cggccatctt ggctcctcct gtaacaattt cttgagatga     420

agatcctgaa taccaaagag ggccgctgac aggtctagga gtacacttct agcacctagc     480

agagagaggc ttcactacat catgcttcct gacatctctc cctttgaaga gcagtcagac     540

tcctgctttg ctcttcagac ttaatttggg ggtttaacag gtgaggttgc tgggggaact     600

cttttacaac atctctctga aagaatccgg gctgccagtt tcatttggtt tgggtgtcag     660

tagcatgatg gaaagacaaa aaaacacaac ttgacatctg cagaaatggg ttcaaatttt     720

acctgcaact caccaattct gtggc                                           745

<210>     8
<211>     879
<212>     RNA
<213>     Homo sapiens (RP11-1L12.3-004 ENST00000530430.1)

<400>     8
aatcagcgag actccgtggg cgtaggaccc tccgagccag gtgcgggata taatctcgtg      60

gtgcgacgct ttttaagccc ttcagaaaag cgcagtgttc gggtgggagt gacccgattt     120

tccaggtgcc gcccgtcacc cctttctttg actcagaaag ggaactccct gaccccttgc     180

gcttcccaag tgaggcaatg cctcgccctg cttcggctcg cgcacggtgc gcgcacccgc     240

tgacctgcac ccactgtctg gcactcctta gtgagatgaa cccggtacct cagatggaaa     300

tgcagaaatc acccgtcttc tgcgtcgctc acgctgggag ctgtagaccg gagctgttcc     360

tattcggcca tcttggctcc tcctgtaaca atttcttgag atgaagatcc tgaataccaa     420

agagggccgc tgacaggtct aggagtacac ttctagcacc tagcagagag aggcttcact     480

acatcatgct tcctgacatc tctccctttg aagagcagtc agactcctgc tttgctcttc     540

agacttaatt tgggggttta acagccatgt gaagtgctca ctcccccttt gccttctgcc     600

atgattgtgt gtttcctgag gcctccccag aagctgagaa gatgcttcct gtacagcctg     660

cagaactgga tagcccagag ctattccatg tattccatgg atgggcacat ttggaagttt     720

ggttctaaaa tctaaaagaa gaaatttaag tgtgctgaag ataactgcaa ctccaaacct     780

aacgctgtgt aattctatgt tctgccattt tgaagcaaag tggaaccaga agtgagagga 840

ctatttctag gtcatgctac ggttaccctg accccagtc 879

<210> 9
<211> 447
<212> RNA
<213> Homo sapiens (CTD-2319I12.1-001 ENST00000566140.1)

<400> 9
cagcaacatg aagttggcag ccttcctcct cctgtgatcc tcatcatctt cagcctagag 60

gtacaagagc ttcaggctgc aggagaccgg cttttgggta cctgcgtcga gctctgcaca 120

ggtgactggg actgcaaccc cggagaccac tgtgtcagca atgggtgtgg ccatgagtgt 180

gttgcagggt aaggacaggt aaaaacacca ggccctccct gctttctgaa acgttgttca 240

gtctagatga agagttatct taaggatcat ctttccctaa gatcgtcatc ccttcctgga 300

gttcctatct tccaagatgt gactgtctgg agttccttga ctaggaagat ggatgaaaac 360

agcaagcctg tggatggaga ctacagggga tatgggaggc agggaagagg ggttgtttct 420

tttaataaat catcattgtt aaaagca 447

<210> 10
<211> 630
<212> RNA
<213> Homo sapiens (CTD-2319I12.1-002 ENST00000590346.1)

<400> 10
gggggaagaa gaaaagagga tgccagtcct tgttcctagg ccaagacctg agccctgtaa 60

tattaatatt tggatctaaa atcagccttc caacagcaac atgaagttgg cagccttcct 120

cctcctgtga tcctcatcat cttcagccta gaggtacaag agcttcaggc tgcaggagac 180

cggcttttgg gtgtctgacc cttctcaaca cagcatcaga cagccacctc tattgactga 240

agttgacaat ggagaataaa actcattacc cctgctgaat ccaggggccc ctttttacag 300

atgagaaaag ttccagaaag acccagagac ataatcaggt acctgcgtcg agctctgcac 360

aggtgactgg gactgcaacc ccggagacca ctgtgtcagc aatgggtgtg gccatgagtg 420

tgttgcagga tgaagagtta tcttaaggat catctttccc taagatcgtc atcccttcct 480

ggagttccta tcttccaaga tgtgactgtc tggagttcct tgactaggaa gatggatgaa 540

aacagcaagc ctgtggatgg agactacagg ggatatggga ggcagggaag aggggttgtt 600

tcttttaata aatcatcatt gttaaaagca 630

<210> 11
<211> 630
<212> RNA
<213> Homo sapiens (CTD-2319I12.1-003 ENST00000587298.1)

<400>    11
gggggaagaa gaaaagagga tgccagtcct tgttcctagg ccaagacctg agccctgtaa    60

tattaatatt tggatctaaa atcagccttc caacagcaac atgaagttgg cagccttcct    120

cctcctgtga tcctcatcat cttcagccta gaggtacaag agcttcaggc tgcaggagac    180

cggcttttgg gtgtctgacc cttctcaaca cagcatcaga cagccacctc tattgactga    240

agttgacaat ggagaataaa actcattacc cctgctgaat ccaggggccc ctttttacag    300

atgagaaaag ttccagaaag acccagagac ataatcaggt acctgcgtcg agctctgcac    360

aggtgactgg gactgcaacc ccggagacca ctgtgtcagc aatgggtgtg gccatgagtg    420

tgttgcagga tgaagagtta tcttaaggat catctttccc taagatcgtc atcccttcct    480

ggagttccta tcttccaaga tgtgactgtc tggagttcct tgactaggaa gatggatgaa    540

aacagcaagc ctgtggatgg agactacagg ggatatggga ggcagggaag aggggttgtt    600

tcttttaata aatcatcatt gttaaaagca    630

<210>    12
<211>    474
<212>    RNA
<213>    Homo sapiens (CTD-2319I12.1-004 ENST00000592556.1)

<400>    12
gtgtttttcc aaagccttcc aacagcaaca tgaagttggc agccttcctc ctcctgtgat    60

cctcatcatc ttcagcctag aggtacaaga gcttcaggct gcaggagacc ggcttttggg    120

ggcccctttt tacagatgag aaaagttcca gaaagaccca gagacataat caggtacctg    180

cgtcgagctc tgcacaggtg actgggactg caaccccgga gaccactgtg tcagcaatgg    240

gtgtggccat gagtgtgttg cagggtaagg acagatgaag agttatctta aggatcatct    300

ttccctaaga tcgtcatccc ttcctggagt tcctatcttc caagatgtga ctgtctggag    360

ttccttgact aggaagatgg atgaaaacag caagcctgtg gatggagact acaggggata    420

tgggaggcag ggaagagggg ttgtttcttt taataaatca tcattgttaa aagc    474

<210>    13
<211>    561
<212>    RNA
<213>    Homo sapiens (CTD-2319I12.1-005 ENST00000590012.1)

<400>    13
gtttttccaa agccttccaa cagcaacatg aagttggcag ccttcctcct cctgtgatcc    60

tcatcatctt cagcctagag gtacaagagc ttcaggctgc aggagaccgg cttttgggtg    120

tctgaccctt ctcaacacag catcagacag ccacctctat tgactgaagt tgacaatgga    180

gaataaaact cattacccct gctgaatcca ggggcccctt tttacagatg agaaaagttc    240

cagaaagacc cagagacata atcaggtacc tgcgtcgagc tctgcacagg tgactgggac    300

tgcaaccccg gagaccactg tgtcagcaat gggtgtggcc atgagtgtgt tgcagggtaa 360

ggacagatga agagttatct taaggatcat ctttccctaa gatcgtcatc ccttcctgga 420

gttcctatct tccaagatgt gactgtctgg agttccttga ctaggaagat ggatgaaaac 480

agcaagcctg tggatggaga ctacagggga tatgggaggc agggaagagg ggttgtttct 540

tttaataaat catcattgtt a 561

<210> 14
<211> 558
<212> RNA
<213> Homo sapiens (CTD-2319I12.1-006 ENST00000589987.1)

<400> 14
atcctcatca tcttcagcct agaggtacaa gagcttcagg ctgcaggaga ccggcttttg 60

gatactgcta atcagtccta gcactctttc tctttgaacc tggacaaggt gtctgaccct 120

tctcaacaca gcatcagaca gccacctcta ttgactgaag ttgacaatgg agaataaaac 180

tcattacccc tgctgaatcc aggggcccct ttttacagat gagaaaagtt ccagaaagac 240

ccagagacat aatcaggtac ctgcgtcgag ctctgcacag gtgactggga ctgcaacccc 300

ggagaccact gtgtcagcaa tgggtgtggc catgagtgtg ttgcagggta aggacagatg 360

aagagttatc ttaaggatca tctttcccta agatcgtcat cccttcctgg agttcctatc 420

ttccaagatg tgactgtctg gagttccttg actaggaaga tggatgaaaa cagcaagcct 480

gtggatggag actacagggg atatgggagg cagggaagag gggttgtttc ttttaataaa 540

tcatcattgt taaaagca 558

<210> 15
<211> 605
<212> RNA
<213> Homo sapiens (CTD-2319I12.1-007 ENST00000588180.1)

<400> 15
tcctcatcat cttcagccta gaggtacaag agcttcaggc tgcaggagac cggcttttgg 60

atactgctaa tcagtcctag cactctttct ctttgaacct ggacaaggtg tctgaccctt 120

ctcaacacag catcagacag ccacctctat tgactgaagt tgacaatgga gaataaaact 180

cattacccct gctgaatcca ggggcccctt tttacagatg agaaaagttc cagaaagacc 240

cagagacata atcaggtacc tgcgtcgagc tctgcacagg tgactgggac tgcaaccccg 300

gagaccactg tgtcagcaat gggtgtggcc atgagtgtgt tgcagggtaa ggacaggtaa 360

aaacaccagg ccctccctgc tttctgaaac gttgttcagt ctagatgaag agttatctta 420

aggatcatct ttccctaaga tcgtcatccc ttcctggagt tcctatcttc caagatgtga 480

ctgtctggag ttccttgact aggaagatgg atgaaaacag caagcctgtg gatggagact 540

acaggggata tgggaggcag ggaagagggg ttgtttcttt taataaatca tcattgttaa 600

aagca                                                                 605

<210>    16
<211>    466
<212>    RNA
<213>    Homo sapiens (CTD-2319I12.1-008 ENST00000589777.1)

<400>    16
tactgttttg agattatgtg aatgcattgc ttttaactag aatatataag atgttaatga      60

tattattctt caagtaataa atcatattgc atattgaatg aaaagaaaaa tgtagaactg     120

acttcttaat caagggctgt tctccctagc atctgtccca aaagtacggt ggttttcagc     180

acatccgttc ttccataggt acctgcgtcg agctctgcac aggtgactgg gactgcaacc     240

ccggagacca ctgtgtcagc aatgggtgtg gccatgagtg tgttgcaggg taaggacaga     300

tgaagagtta tcttaaggat catctttccc taagatcgtc atcccttcct ggagttccta     360

tcttccaaga tgtgactgtc tggagttcct tgactaggaa gatggatgaa aacagcaagc     420

ctgtggatgg agactacagg ggatatggga ggcagggaag aggggt                    466

<210>    17
<211>    2036
<212>    RNA
<213>    Homo sapiens (LINC00330-001 ENST00000414562.1)

<400>    17
aaacagttga gaacagactc caccttgtag gacagtggca tttctgaaag ttcagaacaa      60

ttccccgagc aactttacct cacaatctca ttggaacggc tgcatctgtg gggccctggg     120

agagactcag cagcagacag ggtctccaga gcacacgaca agaggaagaa tgcatgaagg     180

tccaccggca gtcgaatcag ccaaagcaca agacaaacac aaagtcttgc tctgtcgccc     240

aggctggagt gcagtggtgc gaccttggct cactggtttg gggcatgttc ctgggtgtgt     300

ctgtgaggag caagaaagcc agttcaagtc ccaaaacctc aaaagtaggg aagccgacag     360

ggcagcctta agtctgtggc tgaaggcctg agagaccctg gcaaatcact gatataagtc     420

caaaagctga agaacttgga atctgatgtt ggaatctgat cttggcccat ccttgaagaa     480

tctgggggta ataaataccc tgaacattga tgaacccaaa gggaagccag aaggtcagat     540

gtcagctgtc gggaagaaag gctggatagg aaataggaaa aagcagggca aacaaggagc     600

ttcaaggact gaaaccctta agaaccactt gactctgtct ctcaccacct ccagccttga     660

tgacacggtg acatatagaa gaactgatgc cctttgcttt gagctgcaca aaaacctggc     720

gctggacttg gagaagctga aagacaggac ccggcaggag cctgaggagc tgcaggctgc     780

tagcctgtag accaccgtgc tgtgttagcc actgcagctg ggaccattct gacactcaga     840

gcctggaaat ggctgctccc tcctgcctgc tatccaagta tcctgtggat acttggcttt     900

tctcttgtgg gcaacactaa cctgaaactc tttggggaag ggaattgagg aaatgtagtt 960

ccaagattag ccaatttaac tcagcataaa cccatcacag ggtttgacta cagagttata 1020

acgttattct gtaggcaatg aaaacagaag tttttgaaaa aggcatcaat ttgatgaaat 1080

tgatattttg gggaagatta atcttctgct gagtaaaata taacaggttg gaggaagatg 1140

aaaatggaga gagtagagtg taattaaaag tttgttggag gccgggcacg ggattacggc 1200

tcactcctgt aatcccagca ctttgggagg ctgaggtggg cggatcacct gaggtcagga 1260

gcttgagatc atcccggcca acatagtgaa accctgtctc taataaaaat acgaaaaatt 1320

agctgggcat ggtggcgcac acctgtaatc tcagctactc gggaggctga ggcaggagaa 1380

tcccttgaac ccaggaggca gaggttgcaa tgagccaaga tggtgtgatt gcactccagc 1440

ccaggggaca atgtgagact ctgtctcaaa aaaaaaaaaa aatttgttgt agtagtccag 1500

gtgtccgggt gttgttacca gggcacccat gatgaaataa ctgggagagt ccttttttct 1560

gactggtttt cttcagtccc tttatctaca ggcacaactg ctgaagaaac cagatggcct 1620

gggatggcac cagagctttt ttacccttga ccagatacta gagggaatta agaccccaca 1680

agtgggcacg aactggaact ggcgacccct agttgccttc agatcattaa cacatcatta 1740

taatgctaaa attccctgcc attttgtgaa catgggttgc atgaagacgt aagtttatga 1800

attgcctctg cacacctaga gtcccaccct gtacaggcta acattcctcc ctacatgcac 1860

ccccccacaa acacaccctg cctcccccag tccttagaaa ccctatgcct ggccgggcgc 1920

agtggctcac gcctgtaatc ccagcacttt gggaggctga ggcgggcgga tcactaggtc 1980

aggagatcga gaccatcctg gctaacacgg tgaaaccctg tctttactaa aaatcc 2036

<210> 18
<211> 742
<212> RNA
<213> Homo sapiens (RP11-114H23.2-001 ENST00000548702.1)

<400> 18
cctggcctcc agcttctgtc tgcttattcc tgggatgttg ttaagagcac cactcagccc 60

ctcagcccct aagcagcctc ttccagtatt agccagtgcc ccaggaaaga tgtatcatct 120

ttcttgctgc ccttgaagag tggtgtccag tgaatctttc aaagaatgat actgagcttc 180

attgttgatg aacaaaaatg caaatctcac ccagagcatg caggaacatg ctataatcac 240

tacactccta gaatgtgatc tctttcaata ttgactgcat ccatgccttc cacacacttg 300

caccagttgg gaggatggag atgtcatctg ggcagaggga gaagagcagt accattcaga 360

ggtctgtccc tcttaagata tattctctct gtcaacctag accatagttg ttcaacataa 420

tacttacaca tttgaaaaaa gaagattgtt ttattggagt gccttaagat gttttcaaca 480

tatggatttt acagcagggc agttcaagag gcatgaggaa tgtttctttt ctctatttgt 540

tggagcactg gagtgtgagc tgaagcagcc taatcctgac ttgcagaaat ggagaggaca 600

tgacttaccg gaattgttca tggaattggt ttggctaatt ctcattgatt ctgccatttc 660

tccagaagcc attactacag ctgttgaaac aggggtagag aaaaatgaca ggtgctaagt 720

ggtatctagg aggttaataa tg 742

<210> 19
<211> 6675
<212> DNA
<213> Homo sapiens (HERES gene)

<400> 19
ggcaaccagc ttgcgtccgt ttccatgttg tgggagattt gttctttcgc tctttgtggt 60

aaatcttgct actgctcact ctttgagtcc acactgcttt tatgagctgt aacactcacc 120

gggaaggtct gcagcttcaa tcctgaagcc agcgaggcca ccagcccacc gggaggaacg 180

aacaactcca gacgcaccgc cttaagagct gtaacactca ccgcgaaggt ctgcagcttc 240

actcctgagc cagcgagacc acgaacccac cagaagaaag aaactccgaa cacatccgaa 300

caccagaagg aacaaactcc agacgcgcca actgtaacac tcaccgcgag gatccgtggc 360

ttcattcttg aagtcagtga gaccaagaac ccaccaattc cggacacact gtgagtcagg 420

atcagtctta ttatagctaa ttagagcaac cccattgcta aagagtttag agcctgggtc 480

agacttcatg gtagcaaagc aatgttttat tgtaatagtg taattaggaa tgtgtggcaa 540

tattttaacc cctcctccca agagttccac aatgctttct taacatgttt ataaatattt 600

gcttttgtgc cattttaggg gagctttccg tacaaacatt tggcttttaa tgagtcatcc 660

acactctgat tcatacccat aagcttcctg ctttgttatc agtgaatatt gggctcagta 720

actgccttac ttttataatt tacatgaatg aatatggaaa aggtggtgga ggtagaaaga 780

catattcagg taaatataaa aacaccctgg tttctttaca agagattgac aaatacggcc 840

caatacccta tcatttaaag taagtgccag aattgtgatg gatttgtgtt tggccttaaa 900

aaaaaataaa aataaaaata aaaacaaaaa cacttggata attctacctg atgttacatt 960

tttctttttt tttttctttt cttttttttt tttttttttt gagatggagt tttcctctgt 1020

cacccaggct ggagtgcagt gacgtgacct cagctcgctg caacctctgc ctcccagatt 1080

caagcaattc tctctcaaat tgctcagaag ctgagcaatt ctgcctcagc ttcccaaata 1140

gctgggatta caggtgcgtg ccaccaggct cagctaattt ttgtattttt agtagagatg 1200

gggtttcacc atgttggcca ggctggtctt gaactcccga cctcaggtga tacgcccgcc 1260

tcggcctccc aaagtgctgg gattacaggc atgagccact gcgcccagcc tgatgttcca 1320

tttttctaat caccaagtgt atttattagc taagggtatc ccagaaatta aattaccaac 1380

ataaaatttt acttgtgcta ttataattta gctattaaat cctaaaatta tcaactgaaa 1440

gctactggaa aaataaatac ttctctttct tttctccata ttgaaagtaa agagaatgag 1500

gatccttagc taagatttta attaaatctt ttctgagcca gaattgcaaa gcttttcttt 1560

gtataaataa ggactcgagt tataaagcat agaactagca atgagtctat cttggtccat 1620

ttggattgct ataaaggagt acctgaggct gggtaattta taaagaaaag acgtttattc 1680

gacacatgat tctgcaggat gtacaagaag catggcacca gcatctgctc ttggtgaaaa 1740

cttcaggtgg ctttgactcc tggtggaaga agggaagtct agcttgtgca gagctcccat 1800

ggccagagaa ggaagggggg tttgaggagg tgccaggctc tttgaataga gcgagaactc 1860

actcattacc aagagaaggc accaagccat tcatgaggga tacacccctg tgacccaaac 1920

cttgcccccc ttccaacatt ggggcttaaa tttcaatgtg atgtttaggg gtacaaacat 1980

ccaaaccaca gcagagaaat acagagttct gtatttctat tcccaaaggg caaaatttgg 2040

gaggtgaatg ggtctatatg tgtgttgggg ggaagaagtt aatatgaaga aaagaaacta 2100

aagtgcttta cataataatg agaattacag attaaaaaat ctgtaatctt tccctaaaaa 2160

gggaaagaag taccatgcaa attatgaggt aagtagacta tatgacttat ttatttttat 2220

taggttagag caagaattgc aaactcaagg tcctgactcc ttgaattgca ctctatggac 2280

ccagtcatac ttcatttgca tattatatat taaatgcttt ggcatgatct ttgctccccc 2340

ttacatcctc aaaacactct ttttccattt ttcttaaaat atatgttcct ctttgtctat 2400

attttatttt tccaactttg aaaaggactt acacataaaa atggtatcac tttcttctta 2460

actgtaagaa aaatggtctc agccatacat tgatgaatgg cagctgtcat tcagcctcag 2520

tgtgaaggag acaacaatga gtggtgagga ctgtaataaa atggaaagca catgcccatc 2580

caaaagtgag cattcacttc taggaactag taaaaatggt aatagccaga tctttctatt 2640

ttttcacaag aagcctgaaa gtatttaaat attttatgac taattcaaat gctttaaaaa 2700

taagcaattt gattctagga ggccaaacta gttgctatct atgatttaaa ccatagtgtc 2760

tttcagaatt aatgtatgtc cttggcgggt gtgtgtgtgt gtgtgtgtgt gtgatcccaa 2820

aacaccaata attaccagtg aaaaatttag gagattaaat atgtgtactt taatccacat 2880

tgggggaaga gagagatata aaaggtgctg agaaccttga agtggtgccc ctcctgatct 2940

gccttctacc tcttcaacat acgtgctgtg caccatgaaa gacttgaagt gcaatggcaa 3000

tctgtgatcc tttgtggaaa cctttcccta atttgcccca gagtgaatgt ttcacacttg 3060

gattcacatt tgctgagaga ggcggaaaaa tcttagggac aattagaaca ataataacaa 3120

cattcatatt attattcaat gacagcaaca ttcatattca ttctttcatt cagcaaatat 3180

ttattgcaca cttgctatgt gctagaacta taaaatggca atgatgtgtt aggtaatttg 3240

taatcatggg gaaaaaatca gttagggaaa gacaaaactg ccttccctta tagagattat 3300

attcattgag gagacaaata ataaataaaa tgaatagtgt aacataggtt agatagtgac 3360

aagtgctatg gagaaaaaga aagtaggaaa gggtcatttc tctggtttcc tgatgtgtaa 3420

ttggaataga tatccttggt agctggtaga atctctgcag tgattccttg tactgtgggg 3480

taaaagctat gttagcaggg gagaccaaga ggaatagctg aaacaaaatt cactttctca 3540

gtaagatagt aaataagaaa gtgatattgc atcctgggaa acatggccga gattagtgct 3600

tctctttaag acctaaagga tgcagagtga tggccctcac tcatcctcat ttaattcacc 3660

tgtcaggccc ttgcaaaaat tggtctgatc ctgggggata ataaactcaa ccaaatagtt 3720

gccccaattg cttttgttat gcgagatata tctttgctag aacaaactga ttaacctggc 3780

ctcaggtacc ttgcattgat tggcaaatgt attcatttct attcctgtaa agaaagagaa 3840

acaaaagcac gggaatgaca cagactttta cagcactgag cagggctaat tctcatgccc 3900

tctgtcataa tataacctaa agggacttgg actgtctgga tactccgcac atcaacacca 3960

taacccacta tactgatgat atcatgtgtg atggttttca aatatgcaca caaatttctg 4020

attcttcttt cttcaagagg tggagcttaa ttccccttca attgagggtg agtcgggcat 4080

agtgacttgc ttctaattaa taaaatattg cagaagtgat gatgtggttt ttctaagaca 4140

ctgcaacttc ctttccttgc tctctcttga ataacatgct ctgaaggaaa tcaggtacta 4200

tgtgatgagg acattcaagc aatcctaggg agagattcat gtagaaaaga actaaggcct 4260

cctgccaaaa gccatttgag tcagccctct tggaagtaaa ccctctaccc ctagtaaagc 4320

cttcggatga ctgcagccct gggcagcatc ttgactgcaa actcatgaga gaccctgagc 4380

cacaatcacc cagctaagcc actcttaaat tcctgtccta tactgtgaga taataaatgt 4440

ttactggttt cagctgaaag gtgacagagt gggagtcatg tcaccatctc tggctgtaca 4500

cggaagaatt acagtgagtg acctttaaca caacccctta gtgctccttc ggtacccagt 4560

aaatgagggt tatcatacaa ctatgattat agagcaatag atctggctta ttcatgagac 4620

acttctgagc agtcataaat taaagcatgc atgcaaaagc ccaagatatg ccgttaacaa 4680

cactggaacc agacctcggt tttctcactt tattatctgt tctcctcttc tttggtttct 4740

ttttattttg tttttttcca tcattgaatg atggtgaagt taataatgga aaagagggag 4800

ggaagaagag aagaaaggaa gttgaatata tgaaggacca aagagaatat taccttcctc 4860

cttctcgggt gtgcttagac acaaaaccta acatccaaat aaaccaaggt gtcatttgga 4920

tctctaatgg agattgccaa gagttctgta tgagacagat tcctattaaa gtctcagaac 4980

acgttctgtg caagatcatc tctggagagc atatatgttg ggaaaccctt ttccctctgc 5040

cttccctaaa tttggtctgg aacactgttt tggggtccac acatatcctt caacccaaca 5100

caatctcaac acaagaagag atcagggtaa atggggatca ggaaggaata tgtatccttc 5160

ttccagtcat gaatgaactt agatatcagg gggacaaatt tctgagtgtt atttctccat 5220

ggcttcccct gatgtggcct aagtgggtga ggaggcattt ttcccagccc ttatcaccca 5280

agtctttcta ttctggcccc tcagatagag acttggtaat acatcacttt tgttttggct 5340

gccctgcctg acccacctct cctctttcta acagaaaata gctgctgccc cactcctgtg 5400

ccaaaatctg ctccaaggga tcctatccta gtcagaaggc tgtactctga atgctgatat 5460

tagctacaac accaaggctt cccactatac aaactggatc ctcaaagtca tccacaccaa 5520

atgaactctc tattctttct cccagtagcc tgagaactgg gtttaccagc cttggccctt 5580

cctttccttc tagcctcaaa acaggatggg aacagagtct ttggctgtga gaatgggctc 5640

aataacaaaa taaagatgac agaagagtca gtgacttcaa tgacagagcg atagaaatta 5700

ccaatataaa cggagagaaa agagattttt taaaaatgaa caaatcccca aggacctgtg 5760

agacaataac tgtgttaggt tttctaaact tacaagtgtt tttatttata tatggtaagc 5820

aactatgcat tatttgtgtc agaattattt tttgtatata tttaaggtat acaacatgat 5880

gtctcaacat acttatatgt agtgaaatgg ttactacagt caccacactt attgtctcac 5940

aggttattgt cttcagaagg agagtaaaaa gaatgctggg tagaagaaat acctgaagaa 6000

ataatggccc aacctcaaat ttggtgaaag acataaactt acagattcaa gaaactgagt 6060

gttccccaaa cagaataaac tttaaagaat ccacactcaa acacatcata atcaaacttc 6120

tgaaaactaa caacaaagaa aaacttaaaa tcagcaagag agaaaccaca cattatcaac 6180

tgatgaaaag tgactcaagt gacagcagat ttataatcag aaacttgaag gacagaagga 6240

agaggcaaaa cattttgaaa gccctaaaag gaaagaactg tcaacccaga attcgacatc 6300

cagtaaaaat atccgtcaag aatgaatatg aaggaaagct aagaatgaat atatcttcta 6360

tttctttgct aaatttttct actttttcat ttgatgtaag agtgtttata agtgcttatg 6420

gagcattttt atgatagttg gtttacaatc cttgtctgag agtagctatg tgagatgacg 6480

tatatagtaa atggtgactg tagtaaccat ttcactacat ataagtatgt caaaacatgt 6540

tgtacacctt aaatatacac aaaaaatagc tctgacacaa aaaatgcata gttgttatta 6600

ccatgtataa ataaaaatac ttgtaagttt agaaaataaa atcattgtct gataattatc 6660

taaataataa agtcc 6675

## Description

### [Technical Field]

The present invention relates to a squamous cell carcinoma diagnostic or prognosis prediction marker and the use thereof and, more particularly, to the use of a long non-coding RNA as a marker composition for diagnosing or predicting the prognosis of squamous cell carcinoma, a diagnostic composition, a diagnostic information-providing method and the like, by means of the correlation between the expression of the long non-coding RNA and squamous cell carcinoma pathogenesis.

### [Background Art]

Squamous cell carcinoma is a carcinoma occurring in squamous cells such as the esophagus, head and neck, and lungs, and has been reported to have a poor prognosis due to a very high metastasis rate, and in particular, esophageal squamous cell carcinoma is known to have a 5 year survival rate of only 5 to 10%.

Meanwhile, squamous cell carcinoma is diagnosed by allowing a patient to drink a contrast medium which is impermeable to X-rays depending on the site of occurrence and confirming the contrast medium adhering to the mucosa with X-rays or by directly observing the site by endoscopy. However, since the diagnostic method can mainly observe only the cell surface, it is difficult to determine the degree or depth of cancer progression. Further, since it is difficult to feel symptoms of squamous cell carcinoma at the initial stage, it is often diagnosed in a state in which the cancer has progressed.

Therefore, in order to ensure the treatment of cancer, patients with squamous cell carcinoma receive treatment such as radiation therapy and anticancer chemotherapy in advance, and then receive treatment to remove a tissue which is highly likely to develop cancer in advance. In addition, since even a skilled doctor cannot be sure whether the above-mentioned cancer is treated before the surgical result of a patient with the squamous cell carcinoma condition is obtained, an affected part of the patient is excised as much as possible such that patients who have undergone surgery are reported to have a poor quality of life and suffer from sequelae such as complications.

Meanwhile, since squamous cell carcinoma responds differently to anticancer chemotherapy depending on the size and extent of the cancer and the patient's intrinsic factors, an accurate prognosis for each patient needs to be predicted.

Thus, although there is a patent (Korean Patent No. 10-124736) for a marker for diagnosing squamous cell carcinoma, it is still insufficient to meet the above need, and there is a very urgent need for the initial diagnosis of the cancer and the prediction of the prognosis of a patient through non-invasive examination using a marker for diagnosing squamous cell carcinoma.

### [Disclosure]

### [Technical Problem]

The inventors discovered that the expression pattern of a long non-coding RNA is related to squamous cell carcinoma pathogenesis, and confirmed that an expression of a particular long non-coding RNA could lead to a marker for diagnosing or predicting the prognosis of squamous cell carcinoma, thereby completing the present invention.

Thus, an object of the present invention is to provide a marker composition for diagnosing or predicting the prognosis of squamous cell carcinoma, including at least one long non-coding RNA (IncRNA) selected from the group consisting of RP11-114H23.1, HERES, RP11-1L12.3, CTD-2319I12.1, LINC00330 and RP11-114H23.2, and a use thereof.

Furthermore, an object of the present invention is to provide a composition for diagnosing or predicting the prognosis of squamous cell carcinoma and a kit for diagnosing or predicting the prognosis of squamous cell carcinoma, including a preparation capable of measuring an expression level of at least one long non-coding RNA (IncRNA) selected from the group consisting of RP11-114H23.1, HERES, RP11-1L12.3, CTD-2319I12.1, LINC00330 and RP11-114H23.2.

Further, another object of the present invention is to provide an information-providing method for diagnosing or predicting the prognosis of squamous cell carcinoma, the method including: measuring an expression level of a long non-coding RNA (IncRNA) selected from the group consisting of RP11-114H23.1, HERES, RP11-1L12.3, CTD-2319I12.1, LINC00330 and RP11-114H23.2 from a sample of a patient with squamous cell carcinoma and comparing the expression level of the long non-coding RNA with that of a control sample.

In addition, still another object of the present invention is to provide a method for diagnosing or predicting the prognosis of squamous cell carcinoma by a preparation including at least one long non-coding RNA (IncRNA) selected from the group consisting of RP11-114H23.1, HERES, RP11-1L12.3, CTD-2319I12.1, LINC00330 and RP11-114H23.2.

However, a technical problem to be solved by the present invention is not limited to the aforementioned problems, and other problems that are not mentioned may be clearly understood by a person skilled in the art from the following description.

### [Technical Solution]

To achieve the objects, the present invention provides
a marker composition for diagnosing or predicting the prognosis of squamous cell carcinoma, including at least one long non-coding RNA (IncRNA) selected from the group consisting of RP11-114H23.1 (ENST00000552856.1, ENST00000552466.2), HERES, RP11-1L12.3 (ENST00000525302.1, ENST00000526061.1, ENST00000531363.1, ENST00000530430.1), CTD-2319112.1 (ENST00000566140.1, ENST00000590346.1, ENST00000587298.1, ENST00000592556.1, ENST00000590012.1, ENST00000589987.1, ENST00000588180.1, ENST00000589777.1), LINC00330 (ENST00000414562.1) and RP11-114H23.2 (ENST00000548702.1).

As an exemplary embodiment of the present invention, the HERES may include a base sequence represented by SEQ ID NO: 3 or SEQ ID NO: 4.

An exemplary embodiment of the present invention, the present invention provides a use of the composition.

Furthermore, the present invention provides a composition for diagnosing or predicting the prognosis of squamous cell carcinoma, including a preparation capable of measuring an expression level of at least one long non-coding RNA (IncRNA) selected from the group consisting of RP11-114H23.1 (ENST00000552856.1, ENST00000552466.2), HERES, RP11-1L12.3 (ENST00000525302.1, ENST00000526061.1, ENST00000531363.1, ENST00000530430.1), CTD-2319112.1 (ENST00000566140.1, ENST00000590346.1, ENST00000587298.1, ENST00000592556.1, ENST00000590012.1, ENST00000589987.1, ENST00000588180.1, ENST00000589777.1), LINC00330 (ENST00000414562.1) and RP11-114H23.2 (ENST00000548702.1).

As an exemplary embodiment of the present invention, the HERES may include a base sequence represented by SEQ ID NO: 3 or SEQ ID NO: 4.

Further, the present invention provides a kit for diagnosing or predicting the prognosis of squamous cell carcinoma, including the composition.

In addition, the present invention provides an information-providing method for diagnosing or predicting the prognosis of squamous cell carcinoma, the method including: a) measuring an expression level of at least one long non-coding RNA (IncRNA) selected from the group consisting of RP11-114H23.1 (ENST00000552856.1, ENST00000552466.2), HERES, RP11-1L12.3 (ENST00000525302.1, ENST00000526061.1, ENST00000531363.1, ENST00000530430.1), CTD-2319112.1 (ENST00000566140.1, ENST00000590346.1, ENST00000587298.1, ENST00000592556.1, ENST00000590012.1, ENST00000589987.1, ENST00000588180.1, ENST00000589777.1), LINC00330 (ENST00000414562.1) and RP11-114H23.2 (ENST00000548702.1) from a sample of a patient with squamous cell carcinoma; and
b) comparing the expression level of the long non-coding RNA with that of a control sample.

As an exemplary embodiment of the present invention, the HERES may include a base sequence represented by SEQ ID NO: 3 or SEQ ID NO: 4.

As another exemplary embodiment of the present invention, when the expression level of HERES or RP11-1L12.3, which is the long non-coding RNA, is high, it is possible to predict that the risk of squamous cell carcinoma pathogenesis is high.

As still another exemplary embodiment of the present invention, when the expression level of at least one selected from the group consisting of RP11-114H23.1, CTD-2319I12.1, LINC00330 and RP11-114H23.2, which are the long non-coding RNA, is low, it is possible to predict that the risk of squamous cell carcinoma pathogenesis is high.

As yet another exemplary embodiment of the present invention, the expression level of the long non-coding RNA may be measured by at least one method selected from the group consisting of polymerase chain reaction (PCR), reverse transcription polymerase chain reaction (RTPCR), real-time polymerase chain reaction (Real-time PCR), RNase protection assay (RPA), a microarray, single molecule imaging, RNA FISH and northern blotting.

Furthermore, the present invention provides a method for diagnosing or predicting the prognosis of squamous cell carcinoma by a preparation capable of measuring an expression level of at least one long non-coding RNA (IncRNA) selected from the group consisting of RP11-114H23.1 (ENST00000552856.1, ENST00000552466.2), HERES, RP11-1L12.3 (ENST00000525302.1, ENST00000526061.1, ENST00000531363.1, ENST00000530430.1), CTD-2319112.1 (ENST00000566140.1, ENST00000590346.1, ENST00000587298.1, ENST00000592556.1, ENST00000590012.1, ENST00000589987.1, ENST00000588180.1, ENST00000589777.1), LINC00330 (ENST00000414562.1) and RP11-114H23.2 (ENST00000548702.1).

As an exemplary embodiment of the present invention, the HERES may include a base sequence represented by SEQ ID NO: 3 or SEQ ID NO: 4.

### [Advantageous Effects]

The inventors discovered that the expression pattern of a long non-coding RNA is related to cancer pathogenesis, investigated the correlation between an expression of a particular long non-coding RNA and squamous cell carcinoma pathogenesis and thus confirmed that the long non-coding RNA can be a squamous cell carcinoma diagnostic or prognosis prediction marker. Therefore, the marker according to the present invention can assist management and treatment of a cancer patient by means of diagnosing or predicting the prognosis of squamous cell carcinoma.

### [Description of Drawings]

FIG. 1 shows a flowchart illustrating a process of discovering a marker for predicting the prognosis of esophageal squamous cell carcinoma.
FIG. 2 shows the results of classifying the long non-coding RNA expression pattern according to the present invention and patients with esophageal squamous cell carcinoma.
FIG. 3 shows the results of measuring the stage-free survival rates of patients with esophageal squamous cell carcinoma using HERES, RP11-1L12.3, CTD-2319112.1, RP11-114H23.1, RP11-114H23.2 or LINC00330 as a marker for esophageal squamous cell carcinoma in the patients with esophageal squamous cell carcinoma.
FIG. 4 shows the results of measuring the stage-free survival rates of patients with head and neck squamous cell carcinoma using HERES as a marker for head and neck squamous cell carcinoma in the patients with head and neck squamous cell carcinoma.

### [Modes of the Invention]

Hereinafter, the present invention will be described in detail.

The inventors discovered that the expression pattern of a long non-coding RNA is related to cancer pathogenesis, investigated the correlation between an expression of a particular long non-coding RNA and squamous cell carcinoma pathogenesis, and confirmed that the long non-coding RNA can be a squamous cell carcinoma diagnostic or prognosis prediction marker, thereby completing the present invention.

The present invention provides a marker composition for diagnosing or predicting the prognosis of squamous cell carcinoma, including at least one long non-coding RNA (IncRNA) selected from the group consisting of RP11-114H23.1, HERES, RP11-1L12.3, CTD-2319112.1, LINC00330 and RP11-114H23.2; a composition for diagnosing or predicting the prognosis of squamous cell carcinoma, including a preparation capable of measuring an expression level of a long non-coding RNA; and a kit for diagnosing or predicting the prognosis of squamous cell carcinoma, including the composition.

The RP11-114H23.1 according to the present invention may include a base sequence represented by SEQ ID NO: 1 (ENST00000552856.1) or SEQ ID NO: 2 (ENST00000552466.2), the HERES may include a base sequence represented by SEQ ID NO: 3 or SEQ ID NO: 4, the RP11-1L12.3 may include any one base sequence selected from the group consisting of base sequences represented by SEQ ID NO: 5 (ENST00000525302.1), SEQ ID NO: 6 (ENST00000526061.1), SEQ ID NO: 7 (ENST00000531363.1) and SEQ ID NO: 8 (ENST00000530430.1), the CTD-2319112.1 may include any one base sequence selected from the group consisting of base sequences represented by SEQ ID NO: 9 (ENST00000566140.1), SEQ ID NO: 10 (ENST00000590346.1), SEQ ID NO: 11 (ENST00000587298.1), SEQ ID NO: 12 (ENST00000592556.1), SEQ ID NO: 13 (ENST00000590012.1), SEQ ID NO: 14 (ENST00000589987.1), SEQ ID NO: 15 (ENST00000588180.1) and SEQ ID NO: 16 (ENST00000589777.1), the LINC00330 may include a base sequence represented by SEQ ID NO: 17 (ENST00000414562.1), and the RP11-114H23.2 may include a base sequence represented by SEQ ID NO: 18 (ENST00000548702.1). The characters shown in the parentheses indicate a transcript ID of a transcript described in GRCh37.p13.

The HERES according to the present invention may include a base sequence represented by SEQ ID NO: 3 or SEQ ID NO: 4, and in this case, may also include a base sequence having 70% or more, preferably 80% or more, more preferably 90% or more, and most preferably 95%, 96%, 97%, 98% or 99% or more sequence homology with the base sequence represented by SEQ ID NO: 3 or 4.

Here, an HERES gene encoding the HERES according to the present invention may include a base sequence represented by SEQ ID NO: 19.

"Squamous cell carcinoma", which is the target disease of the present invention, is a malignant tumor derived from keratinocytes of the epidermis. Squamous cell carcinoma is a non-melanoma skin cancer which is more complex than basal cell carcinoma in terms of biological aspects such as metastasis according to tumor size and depth, cause, anatomical location, and histological characteristics, and is one of the most commonly occurring skin cancers along with basal cell carcinoma in Korea. Further, squamous cell carcinoma occurs not only on the skin but also on the mucous membranes, its symptoms vary depending on the site of occurrence and the cause, and the cancer generally looks like broken flesh with a relatively large and uneven shape, and since the surface of the tumor (carcinoma) becomes weak at the onset of squamous cell carcinoma, infection by general bacteria is likely to occur, and although there may be pus or a foul odor, it is reported that there are no other subjective symptoms. Types thereof include squamous cell carcinoma arising from sun-damaged skin, and squamous cell carcinoma arising from cicatrices (scars) and chronic inflammatory diseases, arsenic-induced squamous cell carcinoma, radioactivity-induced squamous cell carcinoma, de novo squamous cell carcinoma or verrucous carcinoma depending on the cause, and include squamous cell carcinoma of the face, neck, arms, and the like, squamous cell carcinoma originating in the lungs, squamous cell carcinoma originating in the esophagus and squamous cell carcinoma originating in the head and neck depending on the site of occurrence, but are not limited thereto.

The "long non-coding RNA (IncRNA)" according to the present invention refers to a long non-coding RNA consisting of 200 nt or more among non-coding RNAs which are transcribed, but are not translated, on DNA sequences. It is known that the expression pattern thereof differs depending on the type of cell. According to recent studies, lncRNAs are emerging as a solution to precise transcriptional regulation according to the gene types that could not be revealed by epigenetics in the past while results that lncRNAs, as well as transcriptional regulators or enzymes such as enzymes for histone methylation, acetylation, phosphorylation and the like, help specific transcriptional regulation for each cell are being produced. Such lncRNAs have been reported to contribute to such transcriptional regulation through mechanisms such as inducing a specific protein to a specific DNA region by themselves or being attached to a specific protein to change the activity thereof.

The "preparation" which measures the level of the long non-coding RNA according to the present invention is preferably an antisense oligonucleotide, a primer pair or a probe. The antisense refers to an oligomer having a nucleotide base sequence and a backbone between subunits, in which an antisense oligomer is hybridized with a target sequence in RNA by Watson-Crick base pairing to typically allow the formation of mRNA and RNA:oligomeric heteroduplexes within the target sequence. The oligomer may have exact sequence complementarity or approximate complementarity to the target sequence.

The "kit" according to the present invention may predict the pathogenesis or prognosis of squamous cell carcinoma by confirming the expression level of a long non-coding RNA, which is a marker whose expression is differentially increased in a group of patients with squamous cell carcinoma to confirm whether the long non-coding RNA is over- or under-expressed in a test subject, and by confirming the expression level of a long non-coding RNA whose expression is increased or decreased in a non-patient group to confirm whether the long non-coding RNA is over- or under-expressed in a test subject. That is, the kit may diagnose or predict the prognosis of squamous cell carcinoma by confirming the expression level of a long non-coding RNA and detecting it with a marker. A kit for detecting a marker according to the present invention may include not only a primer or probe for measuring the expression level of a long non-coding RNA which is a marker capable of predicting the presence or absence and prognosis of squamous cell carcinoma, but also at least one other component composition, solution and device suitable for analysis (a gene panel and the like), and the kit for measuring the expression level of a long non-coding RNA which is the marker may be a kit including essential elements required to perform RT-PCR, but is not limited thereto. In addition, the RT-PCR kit may include not only each primer pair specific for a long non-coding RNA which is a marker, but also a test tube or other appropriate containers, a reaction buffer, deoxynucleotides (dNTPs), enzymes such as Taq-polymerase and reverse transcriptase, DNase, a RNase suppressor, DEPC-water, sterilized water, and the like, but is not limited thereto.

In an embodiment of the present invention, six long non-coding RNAs that were significantly correlated with the pathogenesis of esophageal squamous cell carcinoma were selected (see Example 2), and it was confirmed that when HERES and RP11-1L12.3, which are long non-coding RNAs, were highly expressed in a group of patients with esophageal squamous cell carcinoma, the risk of esophageal squamous cell carcinoma pathogenesis was increased, and when CTD-2319I12.1, RP11-114H23.1, RP11-114H23.2 and LINC00330, which are long non-coding RNAs, were lowly expressed, the risk of esophageal squamous cell carcinoma pathogenesis was increased (see Example 3).

The above results confirm that HERES, RP11-1L12.3, CTD-2319I12.1, RP11-114H23.1, RP11-114H23.2 and LINC00330, which are long non-coding RNAs, can be used as esophageal squamous cell carcinoma diagnostic or prognosis prediction markers.

As another aspect of the present invention, the present invention provides an information-providing method for diagnosing or predicting the prognosis of squamous cell carcinoma, the method including a) measuring an expression level of a long non-coding RNA (IncRNA) selected from the group consisting of RP11-114H23.1, HERES, RP11-1L12.3, CTD-2319I12.1, LINC00330 and RP11-114H23.2 from a sample of a patient with squamous cell carcinoma and b) comparing the expression level of the long non-coding RNA with that of a control sample.

In the present invention, when the expression level of HERES or RP11-1L12.3 is high, it is possible to predict that the risk of squamous cell carcinoma pathogenesis is high.

In the present invention, when the expression level of at least one selected from the group consisting of RP11-114H23.1, CTD-2319I12.1, LINC00330 and RP11-114H23.2 is low, it is possible to predict that the risk of squamous cell carcinoma pathogenesis is high.

In the present invention, the expression level of the long non-coding RNA may be measured by at least one method selected from the group consisting of polymerase chain reaction (PCR), reverse transcription polymerase chain reaction (RTPCR), real-time polymerase chain reaction (Real-time PCR), RNase protection assay (RPA), a microarray, single molecule imaging, RNA FISH and northern blotting, but is not limited thereto.

The "information-providing method for diagnosing or predicting the prognosis of squamous cell carcinoma" according to the present invention refers to the provision of objective basic information required to diagnose or predict the prognosis of squamous cell carcinoma as a preliminary step for diagnosis or prediction of the prognosis, and a doctor's clinical determination or findings are excluded.

The "patient sample" according to the present invention includes blood and other liquid samples of biological origin, biopsy specimens, solid tissue samples such as tissue cultures, or cells derived therefrom. More specifically, the patient sample may be a tissue, an extract, a cytolysate, whole blood, serum, and the like, but is not limited thereto.

Hereinafter, preferred Examples for helping the understanding of the present invention will be suggested. However, the following Examples are provided for easier understanding of the present invention, and the contents of the present invention are not limited by the following Examples.

### [Examples]

### Example 1. Experimental preparation and experimental methods

After esophageal tissue excised from a patient with esophageal squamous cell carcinoma was subjected to homogenization, RNA was extracted to perform RNA analysis. For RNA extraction, cell constituents excluding RNA, DNA and proteins were removed using Trizol reagent, and then RNA, DNA and proteins were isolated using chloroform. After an RNA layer was extracted from the separated layer, RNA was washed and deposited. After pellets were made by precipitating RNA, an RNA sample was secured by dissolving the pellets in nuclease-free water. Sequencing was performed from the RNA sample using Illumina Hiseq 2000.

### Example 2. Selection of squamous cell cancer diagnostic or prognosis prediction marker long non-coding RNA

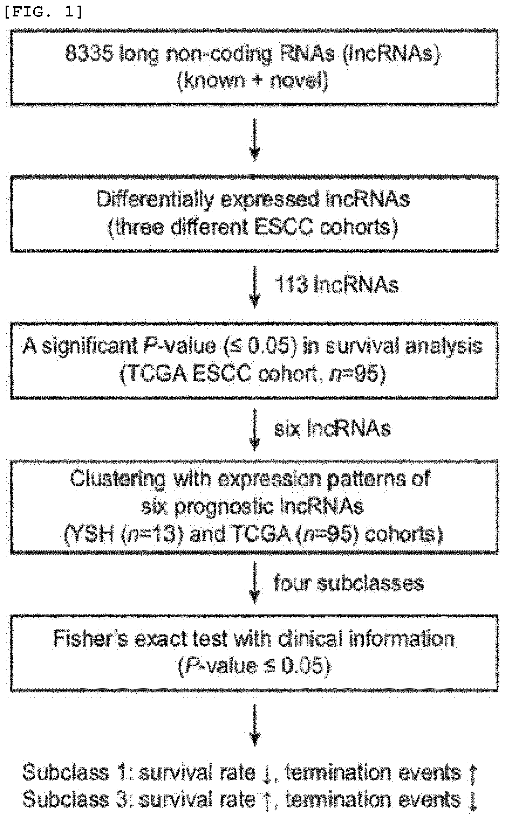

A long non-coding RNA, which is a marker to be used for diagnosing or predicting the prognosis of esophageal squamous cell carcinoma, was selected by the following process as illustrated in FIG. 1.

### 2-1. Confirmation of changes in expression pattern of long non-coding RNA in Korean esophageal squamous cell carcinoma RNA sequencing data

An expression pattern analysis was performed on 8335 long non-coding RNAs using the most commonly used annotation data GENCODE (version 19) and a long non-coding RNA (IncRNA) newly identified in RNA sequencing data of Korean patients with esophageal squamous cell carcinoma.

### 2-2. Selection of long non-coding RNA with commonly changing expression pattern from independent Korean, Chinese and TCGA patient groups

Based on the results in Example 2-1, long non-coding RNAs with a commonly changing expression pattern in Korean, Chinese and TCGA patient groups were selected in order to find long non-coding RNAs specifically over- or under-expressed in patients with esophageal squamous cell carcinoma.

As a result, as illustrated in FIG. 1, 113 long non-coding RNAs were selected.

### 2-3. Selection of squamous cell cancer diagnostic or prognosis prediction marker long non-coding RNA from group of patients with esophageal squamous cell carcinoma of TCGA

Based on the results in Example 2-2, each long non-coding RNA expression pattern and 5 year survival rates of patients were analyzed in a group (n=95) of patients with esophageal squamous cell carcinoma of TCGA including various patient backgrounds.

As a result, as illustrated in FIG. 1, it was confirmed that a total of six long non-coding RNAs (HERES, RP11-1L12.3, CTD-2319112.1, RP11-114H23.1, RP11-114H23.2, LINC00330) showed a significant correlation with the prognosis of patients.

From the above results, it could be confirmed that HERES, RP11-1L12.3, CTD-2319112.1, RP11-114H23.1, RP11-114H23.2 and LINC00330, which are long non-coding RNAs, could be used as esophageal squamous cell carcinoma diagnostic or prognosis prediction markers.

### Example 3. Application of squamous cell cancer diagnostic or prognosis prediction marker

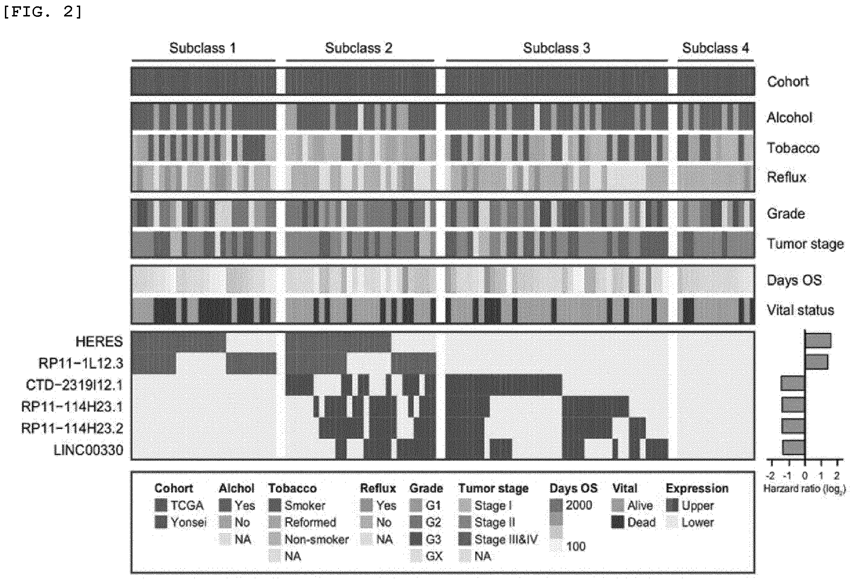

Based on the results in Example 2, as illustrated in FIG. 2, the groups of patients with esophageal squamous cell carcinoma of Koreans (n=13) and TCGA (n=95) were classified into four groups based on the expression pattern of the six long non-coding RNAs (subclass 1, 2, 3 and 4).

Further, a correlation between the patient survival rate of the four groups and the expression level of the above six long non-coding RNAs was confirmed.

As a result, as illustrated in FIG. 2, it was confirmed that the survival rate of a patient group (subclass 1) with high expression of HERES and RP11-1L12.3 and low expression of CTD-2319I12.1, RP11-114H23.1, RP11-114H23.2 and LINC00330 was low, but the survival rate of a patient group (subclass 3) with low expression of HERES and RP11-1L12.3 and high expression of CTD-2319112.1, RP11-114H23.1, RP11-114H23.2 and LINC00330 was high.

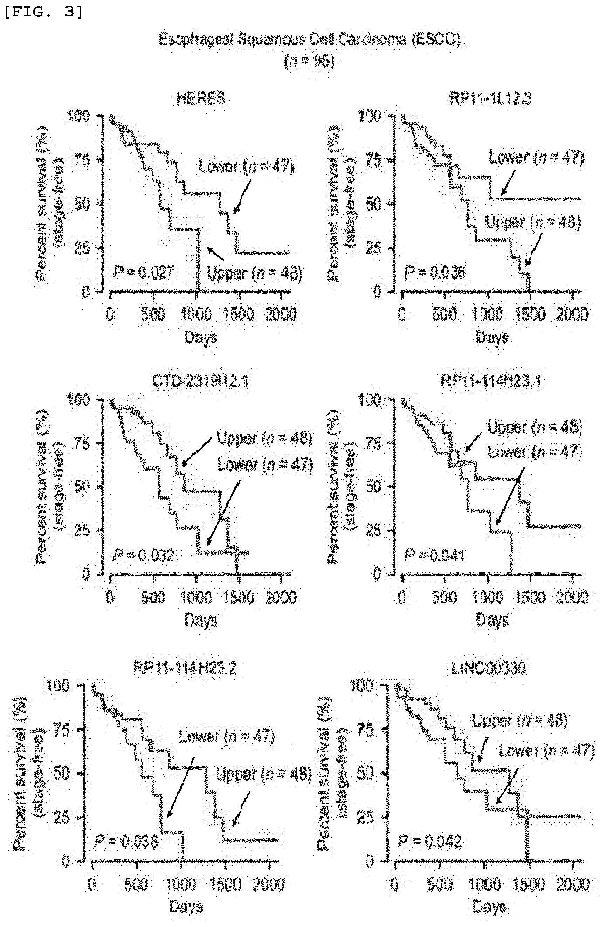

In addition, as illustrated in FIG. 3, stage-free survival rates of patients with esophageal squamous cell carcinoma for 5 years were measured using HERES, RP11-1L12.3, CTD-2319112.1, RP11-114H23.1, RP11-114H23.2 or LINC00330 as a marker in the patients with esophageal squamous cell carcinoma.

As a result, it was confirmed that the lower the expression rate of HERES or RP11-1L12.3 was, the higher the survival rate of patients was, and the higher the expression rate of CTD-2319I12.1, RP11-114H23.1, RP11-114H23.2 or LINC00330 was, the higher the survival rate of patients was.

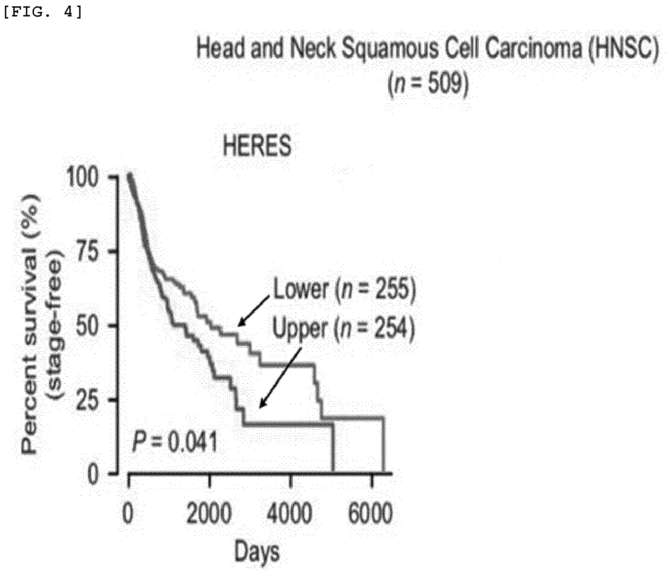

Furthermore, as illustrated in FIG. 4, stage-free survival rates of patients with head and neck squamous cell carcinoma for 5 years were measured using HERES as a marker in the patients with head and neck squamous cell carcinoma.

As a result, it was confirmed that the lower the expression rate of HERES was, the higher the survival rate of patients was.

From the above results, it could be confirmed that the high expression of HERES or RP11-1L12.3, which is a long non-coding RNA, is related to a high risk of esophageal squamous cell carcinoma pathogenesis, and the low expression of CTD-2319I12.1, RP11-114H23.1, RP11-114H23.2 or LINC00330, which is a long non-coding RNA, is related to a high risk of esophageal squamous cell carcinoma pathogenesis.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described embodiments are only illustrative in all aspects and not restrictive.

### [Industrial Applicability]

The present invention is a technology capable of developing a long non-coding RNA which is related to squamous cell carcinoma as a composition capable of diagnosing or observing the prognosis of squamous cell carcinoma using the long non-coding RNA, and since it is possible to more rapidly and precisely diagnose or observe the prognosis of the carcinoma by a non-invasive test compared to diagnostic markers in the related art, the technology according to the present invention can be widely used in the development fields such as the management and diagnosis of the carcinoma.

## Claims

1. A marker composition for diagnosing or predicting the prognosis of squamous cell carcinoma, comprising at least one long non-coding RNA (IncRNA) selected from the group consisting of RP11-114H23.1 (ENST00000552856.1, ENST00000552466.2), HERES, RP11-1L12.3 (ENST00000525302.1, ENST00000526061.1, ENST00000531363.1, ENST00000530430.1), CTD-2319112.1 (ENST00000566140.1, ENST00000590346.1, ENST00000587298.1, ENST00000592556.1, ENST00000590012.1, ENST00000589987.1, ENST00000588180.1, ENST00000589777.1), LINC00330 (ENST00000414562.1) and RP11-114H23.2 (ENST00000548702.1).

2. The marker composition of claim 1, wherein the HERES comprises a base sequence represented by SEQ ID NO: 3 or SEQ ID NO: 4.

3. A composition for diagnosing or predicting the prognosis of squamous cell carcinoma, comprising a preparation capable of measuring an expression level of at least one long non-coding RNA (IncRNA) selected from the group consisting of RP11-114H23.1 (ENST00000552856.1, ENST00000552466.2), HERES, RP11-1L12.3 (ENST00000525302.1, ENST00000526061.1, ENST00000531363.1, ENST00000530430.1), CTD-2319112.1 (ENST00000566140.1, ENST00000590346.1, ENST00000587298.1, ENST00000592556.1, ENST00000590012.1, ENST00000589987.1, ENST00000588180.1, ENST00000589777.1), LINC00330 (ENST00000414562.1) and RP11-114H23.2 (ENST00000548702.1).

4. The composition of claim 3, wherein the HERES comprises a base sequence represented by SEQ ID NO: 3 or SEQ ID NO: 4.

5. A kit for diagnosing or predicting the prognosis of squamous cell carcinoma, comprising the composition of claim 3.

6. An information-providing method for diagnosing or predicting the prognosis of squamous cell carcinoma, the method comprising: a) measuring an expression level of a long non-coding RNA (IncRNA) selected from the group consisting of RP11-114H23.1 (ENST00000552856.1, ENST00000552466.2), HERES, RP11-1L12.3 (ENST00000525302.1, ENST00000526061.1, ENST00000531363.1, ENST00000530430.1), CTD-2319112.1 (ENST00000566140.1, ENST00000590346.1, ENST00000587298.1, ENST00000592556.1, ENST00000590012.1, ENST00000589987.1, ENST00000588180.1, ENST00000589777.1), LINC00330 (ENST00000414562.1) and RP11-114H23.2 (ENST00000548702.1) from a sample of a patient with squamous cell carcinoma; and b) comparing the expression level of the long non-coding RNA with that of a control sample.

7. The information-providing method of claim 6, wherein the HERES comprises a base sequence represented by SEQ ID NO: 3 or SEQ ID NO: 4.

8. The information-providing method of claim 6, wherein when an expression level of HERES or RP11-1L12.3, which is the long non-coding RNA, is high, it is predicted that a risk of squamous cell carcinoma pathogenesis is high.

9. The information-providing method of claim 6, wherein when an expression level of at least one selected from the group consisting of RP11-114H23.1, CTD-2319112.1, LINC00330 and RP11-114H23.2, which are long non-coding RNAs, is low, it is predicted that a risk of squamous cell carcinoma pathogenesis is high.

10. The information-providing method of claim 6, wherein an expression level of the long non-coding RNA is measured by at least one method selected from the group consisting of polymerase chain reaction (PCR), reverse transcription polymerase chain reaction (RTPCR), real-time polymerase chain reaction (Real-time PCR), RNase protection assay (RPA), a microarray, single molecule imaging, RNA FISH and northern blotting.

11. A method for diagnosing or predicting the prognosis of squamous cell carcinoma by using a preparation capable of measuring an expression level of at least one long non-coding RNA (IncRNA) selected from the group consisting of RP11-114H23.1 (ENST00000552856.1, ENST00000552466.2), HERES, RP11-1L12.3 (ENST00000525302.1, ENST00000526061.1, ENST00000531363.1, ENST00000530430.1), CTD-2319112.1 (ENST00000566140.1, ENST00000590346.1, ENST00000587298.1, ENST00000592556.1, ENST00000590012.1, ENST00000589987.1, ENST00000588180.1, ENST00000589777.1), LINC00330 (ENST00000414562.1) and RP11-114H23.2 (ENST00000548702.1).

12. The method of claim 11, wherein the HERES comprises a base sequence represented by SEQ ID NO: 3 or SEQ ID NO: 4.

13. A use of a marker composition comprising at least one long non-coding RNA (IncRNA) selected from the group consisting of RP11-114H23.1 (ENST00000552856.1, ENST00000552466.2), HERES, RP11-1L12.3 (ENST00000525302.1, ENST00000526061.1, ENST00000531363.1, ENST00000530430.1), CTD-2319112.1 (ENST00000566140.1, ENST00000590346.1, ENST00000587298.1, ENST00000592556.1, ENST00000590012.1, ENST00000589987.1, ENST00000588180.1, ENST00000589777.1), LINC00330 (ENST00000414562.1) and RP11-114H23.2 (ENST00000548702.1) for diagnosing or predicting the prognosis of squamous cell carcinoma.

14. The use of claim 1, wherein the HERES comprises a base sequence represented by SEQ ID NO: 3 or SEQ ID NO: 4.
